# EUROPÄISCHE PATENTSCHRIFT

(11) **EP 3 996 768 B1**
(45) Veröffentlichungstag und Bekanntmachung des Hinweises auf die Patenterteilung: **07.05.2025**
(21) Anmeldenummer: 20739938.7
(22) Anmeldetag: 09.07.2020
(51) Int. Cl.: A61M 1/16

(54) **DIALYSEGERÄT MIT DREI BILANZKAMMERN**
DIALYSIS MACHINE WITH THREE BALANCE CHAMBERS
MACHINE DE DIALYSE AVEC TROIS CHAMBRES D'ÉQUILIBRE

(30) Priorität: 09.07.2019 DE 102019118521
(43) Veröffentlichungstag der Anmeldung: 18.05.2022
(73) Patentinhaber: Fresenius Medical Care Deutschland GmbH, 61352 Bad Homburg (DE)
(72) Erfinder: IRRGANG, Tobias, 97633 Aubstadt (DE)
(74) Vertreter: Herrmann, Uwe
(86) Internationale Anmeldenummer: PCT/EP2020/069396
(87) Internationale Veröffentlichungsnummer: WO 2021/005164

(56) Entgegenhaltungen:
- EP-A1- 3 165 243

## Beschreibung

Die vorliegende Erfindung betrifft ein Dialysegerät mit einem Dialysator und mit einer ersten Bilanzkammer und mit einer zweiten Bilanzkammer, von denen jede wenigstens zwei durch eine bewegliche Wand voneinander getrennte erste und zweite Bilanzkammerhälften aufweist, wobei jede erste Bilanzkammerhälfte mit jeweils einem ersten Zulauf und mit jeweils einem ersten Ablauf versehen ist, wobei jede zweite Bilanzkammerhälfte mit jeweils einem zweiten Zulauf und mit jeweils einem zweiten Ablauf versehen ist, wobei die Zu- und die Abläufe jeweils mit Ventilen versehen sind, die ausgebildet sind, den jeweiligen Zulauf bzw. Ablauf zu schließen oder zu öffnen, wobei die ersten Zuläufe der ersten Bilanzkammerhälften mit einer Quelle frischen Dialysats und die ersten Abläufe der ersten Bilanzkammerhälften mit einem Dialysatorzulauf in Fluidverbindung stehen und wobei die zweiten Zuläufe der zweiten Bilanzkammerhälften mit einem Dialysatorablauf in Fluidverbindung stehen und die zweiten Abläufe der zweiten Bilanzkammerhälften mit einem Drain in Fluidverbindung stehen.

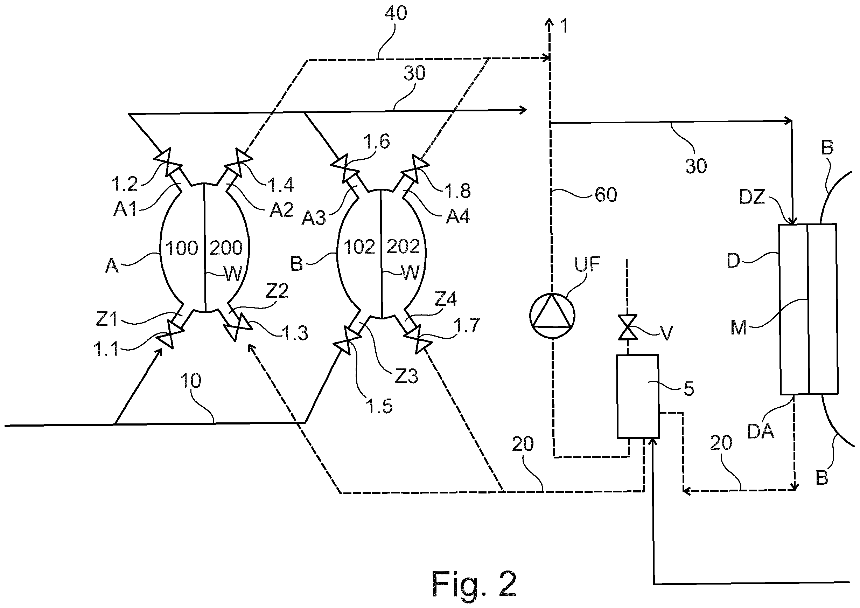

Ein derartiges Dialysegerät ist aus dem Stand der Technik bekannt und in Figur 2 exemplarisch dargestellt.

EP 3 165 243 A1 betrifft Systeme, Methoden und Geräte zur Steuerung des Flüssigkeitsflusses in Behandlungssystemen für Nierenversagen, die drei Bilanzkammer aufweisen.

Figur 2 zeigt ein Dialysegerät im Zustand der "normalen" Hemodialyse ohne Substituatförderung.

Wie dies aus Figur 2 hervorgeht, sind zwei Bilanzkammern A und B vorgesehen, von denen jede eine erste Bilanzkammerhälfte 100, 102 und eine zweite Bilanzkammerhälfte 200, 202 aufweist. Die Bilanzkammerhälften sind jeweils durch eine bewegliche Wand W voneinander getrennt.

Aus Figur 2 ergibt sich des Weiteren, dass jede Bilanzkammerhälfte 100, 200, 102, 202 jeweils einen ersten Zulauf Z1 und Z3 für frisches Dialysat, das über die Leitung 10 zugeführt wird und jeweils einen zweiten Zulauf Z2 und Z4 für gebrauchtes Dialysat, das von dem Dialysator D über die Leitung 20 zugeführt wird, aufweist. Die Zuläufe Z1 und Z2 der ersten Bilanzkammer A sind mittels der Ventile 1.1 und 1.3 verschließbar, die Abläufe A1 und A2 der ersten Bilanzkammer A sind mittels der Ventile 1.2 und 1.4 verschließbar. Die Zuläufe Z3 und Z4 der zweiten Bilanzkammer B sind mittels der Ventile 1.5 und 1.7 verschließbar und die Abläufe A3 und A4 der zweiten Bilanzkammer B sind mittels der Ventile 1.6 und 1.8 verschließbar.

In den Figuren sind die Leitungen, in denen gebrauchtes Dialysat gefördert wird, gestrichelt dargestellt. Leitungen, in denen frisches Dialysat gefördert wird, sind mit durchgezogenen Linien dargestellt.

Die Leitung 30 steht mit den Abläufen A1 und A3 in Fluidverbindung und dient zur Förderung von frischem Dialysat zu dem Dialysator D bzw. zu dessen Zulauf DZ. Dieser ist durch die Membran M in zwei oder mehr als zwei Kammern unterteilt, von denen eine von Dialysat und die andere von Blut B durchströmt wird. Auf der Ablaufseite DA des Dialysators D gelangt das gebrauchte Dialysat einschließlich des Ultrafiltrats in den Sekundärluftabscheider S, der ein Ventil V zur Abfuhr von Luft aufweist.

Von dem Sekundärluftabscheider S wird ein Teil des gebrauchten Dialysats mittels der Ultrafiltrationspumpe UF mittels der Leitung 60 in den Drain 1 gefördert. Der verbleibende Teil des gebrauchten Dialysats gelangt getaktet über die Zuläufe Z2 und Z4 zu den zwei zweiten Bilanzkammerhälften 200, 202 der Bilanzkammern A und B.

Die Leitung 40 steht mit den Abläufen A2 und A4 der zweiten Bilanzkammerhälften 200, 202 in Fluidverbindung und dient zur Förderung von gebrauchtem Dialysat zu dem Drain 1.

Die bilanzierte Förderung des Dialysats gestaltet sich gemäß Figur 2 wie folgt:
Zunächst wird in die erste Bilanzkammerhälfte 100 der ersten Bilanzkammer A über die Leitung 10 Frischdialysat gefördert. Dazu ist das Ventil 1.1 geöffnet und das Ventil 1.2 ist geschlossen. Durch die sich dabei nach rechts bewegende Wand W wird gleichzeitig gebrauchtes Dialysat aus der zweiten Bilanzkammerhälfte 200 der ersten Bilanzkammer A über die Leitung 40 in den Drain 1 entfernt. Dabei ist das Ventil 1.3 geschlossen und das Ventil 1.4 ist geöffnet. Es wird somit genau ein Bilanzkammervolumen gefördert, beispielsweise 30 ml.

Die zweite Bilanzkammer B arbeitet gegengetaktet, d.h. Ventil 1.5 ist geschlossen, Ventil 1.6 ist geöffnet, Ventil 1.7 ist geöffnet und Ventil 1.8 ist geschlossen. Insgesamt wird durch die erste Bilanzkammer A in diesem Takt gebrauchtes Dialysat aus der ersten Bilanzkammer A gefördert, und aus der zweiten Bilanzkammer B Frischdialysat.

Im Anschluss daran erfolgt ein neuer Takt des Bilanzkammersystems, bei dem frisches Dialysat aus der ersten Bilanzkammerhälfte 100 der ersten Bilanzkammer A und gebrauchtes Dialysat aus der zweiten Bilanzkammerhälfte 202 der zweiten Bilanzkammer B gefördert wird.

Der oben beschriebene Ablauf wiederholt sich in stetiger Abfolge.

Es wird stets dieselbe Menge Frischdialysat und gebrauchtes Dialysat gefördert und exakt bilanziert.

Aufgrund der Entwässerung des Patienten entsteht auf der Seite des gebrauchten Dialysates zusätzlich Ultrafiltrat, das durch die Pumpe UF gefördert wird, die sich in der Leitung 60 befindet.

Bei einer Behandlung entstehen ca. 2l Ultrafiltrat pro Patient und einer Behandlungsdauer von ca. 4h.

Die Ultrafiltratmenge wird über die Ultrafiltratpumpe UF gesteuert, die das Ultrafiltrat aus dem Blut des Patienten abzieht.

Die aus dem Stand der Technik bekannte Anordnung gemäß Figur 2 sorgt für eine exakte Kontrolle der Dialysatflüssigkeitsmengen (frisch und gebraucht) und der Ultrafiltratmenge. Die Steuerung der Ultrafiltratmenge ist wichtig, weil eine Mindestmenge zur Vermeidung einer Überwässerung des Patienten gewährleistet sein muss und andererseits eine zu schnelle Entwässerung zu einem Kreislaufkollaps führen kann.

Gemäß dem Stand der Technik kann vorgesehen sein, dass eine zusätzliche Dialysatmenge bzw. Flüssigkeitsmenge über eine separate, nicht dargestellte Pumpe (Substituatpumpe) zum Patienten gefördert wird. Dies wird erforderlich, wenn eine größere Menge an Ultrafiltrat dem Blut entnommen wird als dem Patienten verschrieben wurde. Dann kann das Substituat (in der Regel) nach dem Dialysator zugeführt werden. Grundsätzlich ist auch eine Zugabe vor oder vor und nach dem Dialysator möglich. Substituatmengen zwischen 20 und 40 l pro Behandlungszyklus sind üblich.

Dieses Verfahren wird HDF (Hemodiafiltration) genannt. Der HDF Modus hat den Vorteil, dass ein erhöhter konvektiver Anteil über die Dialysatormembran befördert werden kann und somit insbesondere die mittelmolekularen Urämtoxine besonders effektiv entfernt werden können, die diffusiv nur im geringen Maße über die Membran hinweg befördert werden können.

Zusätzlich zur Substituatpumpe ist bei bekannten Geräten ein weiterer Filter für das Substituat erforderlich, welcher eine Keimfreifiltration des Substitutes sicherstellt. Dies ist deshalb erforderlich, weil das Substituat direkt in das Patientenblut infundiert wird.

Eine solche Anordnung ist somit kompliziert und teuer.

Der vorliegenden Erfindung liegt somit die Aufgabe zugrunde, ein Dialysegerät der eingangs genannten Art dahingehend weiterzubilden, dass dieses einen möglichst einfachen Aufbau aufweist.

Diese Aufgabe wird durch ein Dialysegerät mit den Merkmalen des Anspruchs 1 gelöst. Danach ist vorgesehen, dass eine dritte Bilanzkammer vorgesehen ist, die über zwei Bilanzkammerhälften verfügt, die durch eine bewegliche Wand voneinander getrennt sind, wobei jede der Bilanzkammerhälften der dritten Bilanzkammer jeweils über einen Zulauf und über einen Ablauf verfügt, die jeweils mit Ventilen versehen sind, die ausgebildet sind, den jeweiligen Zulauf bzw. Ablauf zu schließen oder zu öffnen, wobei die beiden Zuläufe mit einer Quelle frischen Dialysats/Substituats in Fluidverbindung stehen und wobei die beiden Abläufe mit dem Dialysatorzulauf in Fluidverbindung stehen.

Die dritte Bilanzkammer wird somit nicht mit gebrauchtem Dialysat beaufschlagt. Stattdessen dient diese ausschließlich zu Förderung frischen Dialysats bzw. Substituats.

Die dritte Bilanzkammer kann derart angesteuert sein, dass diese zeitlich versetzt oder gleichzeitig zu der ersten und zweiten Bilanzkammer arbeitet. Der gleichzeitige Betrieb bringt den Vorteil mit sich, dass die Bilanzierung und somit der Betrieb des Dialysegerätes fortgesetzt werden kann und nicht unterbrochen werden muss, wenn mittels der dritten Bilanzkammer Substituat, d.h. vorzugsweise frische Dialyselösung dem Dialysator zugeführt wird.

Die dritte Bilanzkammer dient somit zur Förderung frischen Dialysats bzw. Substituats zu dem Dialysator, so dass in einer vorteilhaften Ausgestaltung der Erfindung auf eine separate Substituatpumpe verzichtet werden kann.

Die kontinuierlich oder diskontinuierlich von der dritten Bilanzkammer geförderte Flüssigkeitsmenge (z.B. 1 Hub, 30 ml) dient als Substituat für den Patienten. Gleichzeitig wirkt die Membran des Dialysators als Sterilfilter für das Substituat, so dass in einer bevorzugten Ausgestaltung der Erfindung weder eine eigene Substituatpumpe noch ein eigener Sterilfilter vorhanden sind. Abgesehen davon wird kein eigener Schlauchsatz für das Substituat benötigt.

Durch Hin- und Herschalten der Ventile der dritten Bilanzkammer kann nun jeweils ein Volumen einer Bilanzkammerhälfte als Substituat in den Systemkreislauf gefördert werden.

Vorzugsweise stehen die beiden Zuläufe der dritten Bilanzkammer mit derselben Quelle frischen Dialysats in Fluidverbindung, wie die ersten Zuläufe der ersten und zweiten Bilanzkammer, d.h. die Bilanzkammern werden aus derselben Quelle mit frischem Dialysat gespeist. Grundsätzlich ist es auch denkbar, dass die Zuläufe der dritten Bilanzkammer aus einer anderen Quelle gespeist werden, als die ersten Zuläufe der zweiten und dritten Bilanzkammer, so dass beispielsweise für das Substituat eine andere Lösung verwendet werden kann als für das Dialysat.

In einer weiteren Ausgestaltung der Erfindung stehen die beiden Abläufe der dritten Bilanzkammer mit derselben, zum Dialysatorzulauf führenden Leitung in unmittelbarer Fluidverbindung, wie die ersten Abläufe der ersten und der zweiten Bilanzkammer. Die beiden Abläufe der dritten Bilanzkammer münden vorzugsweise in dieselbe Leitung wie die ersten Abläufe der ersten und der zweiten Bilanzkammer.

Weiterhin kann vorgesehen sein, dass das Gerät keine eigene Substituatpumpe zur Förderung einer Substitutionslösung in das Blut des Patienten aufweist. Dadurch vereinfacht sich der Aufbau des Gerätes entsprechend. Wie ausgeführt, übernimmt in diesem Fall die Funktion der Substituatpumpe die dritte Bilanzkammer und die Funktion des Sterilfilters die Dialysatormembran.

Eine oder beide Bilanzkammerhälften der dritten Bilanzkammer können ein kleineres Volumen aufweisen als die Bilanzkammerhälften der ersten und der zweiten Bilanzkammer, um eine besonders präzise Dosierung mittels der dritten Bilanzkammer zu erreichen.

Um eine langsame Dosierung aus der dritten Bilanzkammer zu ermöglichen, kann vorgesehen sein, dass stromaufwärts und/oder stromabwärts der dritten Bilanzkammer ein Drosselorgan angeordnet ist, das ausgebildet ist, den Zustrom und/oder den Abstrom frischen Dialysats aus der dritten Bilanzkammer zu drosseln. Bei dem Drosselorgan kann es sich z.B. um eine Blende, eine Verengung, ein Ventil etc. handeln. Das Drosselorgan kann dahingehend verstellbar sein, dass der Grad der Drosselung veränderbar ist, oder auch nicht verstellbar ausgeführt sein.

Das Dialysegerät kann mit einer Steuerung versehen sein, die ausgebildet ist, die Zufuhr frischen Dialysats zum Dialysator aus der dritten Bilanzkammer über die Behandlungsdauer zeitlich gleichmäßig oder zeitlich ungleichmäßig verteilt durchzuführen. Denkbar ist z.B. eine intermittierende Förderung, eine zeitlich konstante Förderung oder eine zeitlich variable Förderung, z.B. eine profilierte Förderung aus der dritten Bilanzkammer.

Bei dem Dialysator kann es sich beispielsweise um einen High-Flux-Dialysator oder um einen Mid-cut-off-Dialysator handeln. Als High- Flux Dialysatoren werden Filter bezeichnet, die eine Ultrafiltrationrate von 20-70 mL/ m2 * mmHg * h in Humanblut aufweisen. Bei diesen Dialysatoren ist die erfindungsgemäße Substituatzuführung aufgrund der hohen Wasserpermeabilität in Vollblut besonders leicht zu erzielen. Dabei werden bevorzugt Substituatmengen von 5 bis 25 l pro 4-stündiger Behandlung eingestellt, besonders bevorzugt 15 bis 25 I pro 4-stündiger Behandlung.

Besonders effizient wirkt das erfindungsgemäße Dialysegerät bei Mid-cut-off- oder "Protein-leaking"- Dialysatoren. Solche Dialysatoren weisen eine noch höhere Ultrafiltrationsrate in Vollblut auf als High-Flux- Dialysatoren, als nachteilig erweist sich jedoch der erhöhte Albuminverlust, der bis zu 8 g bei einer Behandlung von 4 Stunden betragen kann. Bei Mid-cut- off Dialysatoren findet eine Substituatzuführung durch unkontrollierte Rückfiltration statt. Mit einer erfindungsgemäßen Dialysemaschine kann die Substituatmenge exakt gesteuert werden. Insbesondere sind auch höhere Substituatmengen möglich als mit einer Dialysemaschine gemäß dem Stand der Technik, die mit einem Mid-Cut-off- Dialysator betrieben wird. So kann die optimale Substituatmenge unter Berücksichtigung des erlaubten Albuminverlustes auch bei unterschiedlichen Hämatokritwerten des Patientenblutes bereitgestellt werden. Erfindungsgemäß können Substituatmengen von 5-20 l bei einer 4-stündigen HD- Behandlung eingestellt werden. Bevorzugt sind Substituatmengen von 8 bis 15 l pro 4-stündiger Behandlung.

Die vorliegende Erfindung betrifft des Weiteren eine Steuereinheit konfiguriert zur Durchführung eines Verfahrens zum Betreiben eines Bilanzkammersystems eines Dialysegerätes mit einer ersten Bilanzkammer und mit einer zweiten Bilanzkammer, von denen jede wenigstens zwei durch eine bewegliche Wand voneinander getrennte erste und zweite Bilanzkammerhälften aufweist, wobei jede erste Bilanzkammerhälfte mit jeweils einem ersten Zulauf und mit jeweils einem ersten Ablauf versehen ist, wobei jede zweite Bilanzkammerhälfte mit jeweils einem zweiten Zulauf und mit jeweils einem zweiten Ablauf versehen ist, wobei die Zu- und die Abläufe jeweils mit Ventilen versehen sind, die ausgebildet sind, den jeweiligen Zulauf bzw. Ablauf zu schließen oder zu öffnen, wobei die ersten Zuläufe der ersten Bilanzkammerhälften mit einer Quelle frischen Dialysats und die ersten Abläufe der ersten Bilanzkammerhälften mit einem Dialysatorzulauf in Fluidverbindung stehen und wobei die zweiten Zuläufe der zweiten Bilanzkammerhälften mit einem Dialysatorablauf in Fluidverbindung stehen und die zweiten Abläufe der zweiten Bi-lanzkammerhälften mit einem Drain in Fluidverbindung stehen.

Erfindungsgemäß ist vorgesehen, dass eine dritte Bilanzkammer vorgesehen ist, die über zwei Bilanzkammerhälften verfügt, die durch eine bewegliche Wand voneinander getrennt sind, wobei jede dieser Bilanzkammerhälften jeweils über einen Zulauf und über einen Ablauf verfügt, die jeweils mit Ventilen versehen sind, wobei die beiden Zuläufe mit einer Quelle frischen Dialysats in Fluidverbindung stehen und wobei die beiden Abläufe mit dem Dialysatorzulauf in Fluidverbindung stehen, wobei die Ventile der dritten Bilanzkammer so betrieben werden, dass frisches Dialysat von der dritten Bilanzkammer zu dem Dialysatorzulauf gefördert wird.

Der Begriff des "Dialysats", das mittels der dritten Bilanzkammer gefördert wird, umfasst jedes beliebige Dialysat und Substituat. Es kann sich dabei um dasselbe Dialysat handeln, mit dem die erste und die zweite Bilanzkammer beaufschlagt werden oder auch um eine andere Lösung.

Vorzugsweise werden die Ventile der dritten Bilanzkammer so betrieben, dass sich ein kontinuierlicher Fluss frischen Dialysats aus der dritten Bilanzkammer zu dem Dialysatorzulauf ergibt. In diesem Fall ergibt sich eine kontinuierliche Zufuhr von Dialysat bzw. Substituat zum Dialysator und somit auch zum Patienten über die Membran des Dialysators.

Von der Erfindung ist jedoch auch der Fall umfasst, dass die dritte Bilanzkammer diskontinuierlich betrieben wird.

Um gezielt eine langsame Dosierung von Substituat zu erreichen, kann vorgesehen sein, dass die Zufuhr frischen Dialysats zur dritten Bilanzkammer und/oder die Abfuhr frischen Dialysats aus der dritten Bilanzkammer zu dem Dialysatorzulauf gedrosselt wird.

Es kann eine Auswerte- und Steuereinheit vorgesehen sein, die ausgebildet ist, die dritte Bilanzkammer so zu betreiben, dass die ärztlich verschriebene Substituatmenge zeitlich gleichmäßig auf die Behandlungsdauer verteilt wird. Auch ist es denkbar, dass die Auswerte- und Steuereinheit so ausgebildet ist, dass die ärztlich verschriebene Substituatmenge zeitlich ungleichmäßig auf die Behandlungsdauer verteilt wird. Dabei ist es beispielsweise denkbar, dass die Substituatmenge am Anfang der Behandlung größer ist als am Behandlungsende, dass eine profilierte Zugabe von Substituat erfolgt etc.

Wie ausgeführt ist es von Vorteil, wenn zur Zufuhr von Substituat zum Patienten keine Substituatpumpe verwendet wird. Auf eine solche kann verzichtet werden, da das von der dritten Bilanzkammer geförderte Substituat jedenfalls teilweise über die Membran des Dialysators dem Patienten zugeführt wird.

An dieser Stelle wird darauf hingewiesen, dass die Begriffe "ein" und "eine" nicht zwingend auf genau eines der Elemente verweisen, wenngleich dies eine mögliche Ausführung darstellt, sondern auch eine Mehrzahl der Elemente bezeichnen können. Ebenso schließt die Verwendung des Plurals auch das Vorhandensein des fraglichen Elementes in der Einzahl ein und umgekehrt umfasst der Singular auch mehrere der fraglichen Elemente.

Weitere Einzelheiten und Vorteile der Erfindung werden anhand eines in der Zeichnung dargestellten Ausführungsbeispiels näher erläutert.

Es zeigen:
- Figur 1:: eine schematische Ansicht des Dialysatkreislaufes eines Dialysegerätes gemäß der Erfindung und
- Figur 2:: eine schematische Ansicht des Dialysatkreislaufes eines Dialysegerätes gemäß dem Stand der Technik.

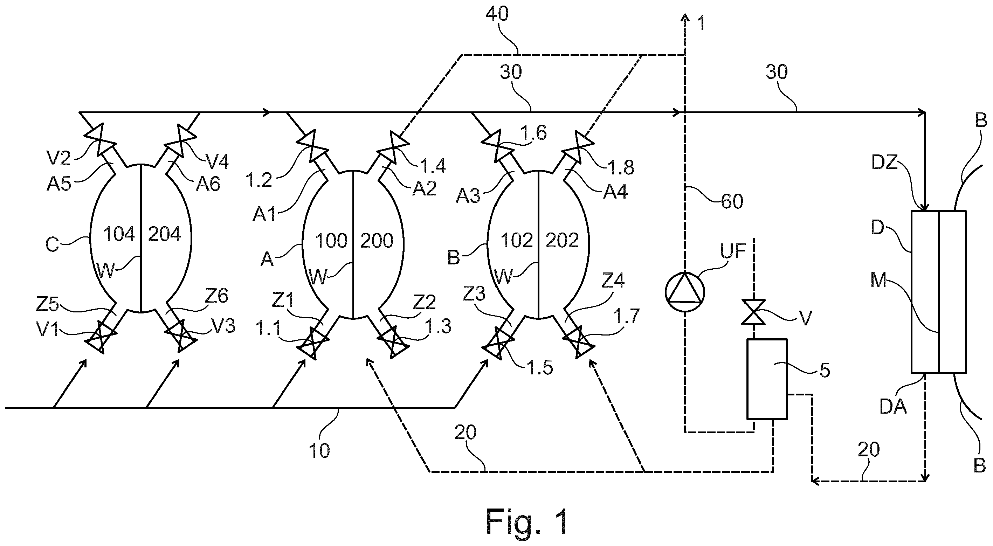

In Figur 1 sind gleiche oder funktionsgleiche Elemente mit denselben Bezugszeichen gekennzeichnet wie in Figur 2, so dass entsprechend Bezug genommen wird.

Der Betrieb des erfindungsgemäßen Dialysegeräts gemäß Figur 1 gestaltet sich wie folgt.

Der getaktete Betrieb der ersten und der zweite Bilanzkammer A, B gestaltet sich wie zu Figur 2 beschrieben, so dass entsprechend auf die dortigen Ausführungen verwiesen wird.

Eine dritte Bilanzkammer C ist parallel geschaltet zu der ersten und der zweiten Bilanzkammer A, B.

Die dritte Bilanzkammer weist zwei Zuläufe Z5, Z6 auf, die mit derselben Leitung 10 in Verbindung stehen, wie die Zuläufe Z1 und Z3 der ersten und der zweiten Bilanzkammer A, B. Im Unterschied zu der ersten und der zweiten Bilanzkammer wird die dritte Bilanzkammer an beiden Zuläufen mit frischem Dialysat aus der Leitung 10 beaufschlagt. Eine Zufuhr von gebrauchtem Dialysat von dem Dialysator zu der dritten Bilanzkammer erfolgt nicht. Dementsprechend fördert die dritte Bilanzkammer aus den Abläufen A5, A6 ausschließlich frisches Dialysat zum Dialysator, das als Substituat für den Patienten dient, indem es zumindest teilweise über die Dialysatormembran M in das Blut des Patienten übertritt. Die Abfuhr frischen Dialysats aus der dritten Bilanzkammer C erfolgt über dieselbe Leitung wie die Abfuhr frischen Dialysats aus der ersten und der zweiten Bilanzkammer. Diese Leitung führt unmittelbar von den jeweiligen Auslässen der Bilanzkammerhälften zum Dialysatoreingang.

Die Betriebsweise der dritten Bilanzkammer entspricht ansonsten der der ersten und zweiten Bilanzkammer, d.h. während bei entsprechender Ventilschaltung eine Bilanzkammerhälfte gefüllt wird, wird durch die dadurch bedingte Verschiebung der Wand W die andere Bilanzkammerhälfte entleert und umgekehrt.

Gleichzeitig wirkt die Dialysatormembran als Sterilfilter für das Susbstituat. Auf einen eigens für die Sterilisation von Substituat vorgesehenen Sterilfilter kann verzichtet werden.

Es besteht der Vorteil, dass keine eigene Substituatpumpe, kein separater Schlauchsatz und kein separater Sterilfilter bereitgestellt werden müssen. Die Funktion der Substituatpumpe übernimmt die dritte Bilanzkammer und die Funktion des Sterilfilters übernimmt die Dialysatormembran.

Auch wird vorzugsweise keine eigene Substituatleitung benötigt, da das Substituat durch dieselbe Leitung zum Dialysator geführt wird, wie das Dialysat, das durch die erste und zweite Bilanzkammer gefördert wird.

Die Vorrichtung kann eine Auswerte- und Steuereinheit umfassen, die die ärztlich verschriebene Substituatmenge gleichmäßig auf die Behandlungsdauer verteilt.

Die Vorrichtung kann eine Auswerte- und Steuereinheit umfassen, die die ärztlich verschriebene Substituatmenge ungleichmäßig auf die Behandlungsdauer verteilt. Das heißt, dass beispielsweise die Substituatmenge am Anfang der Behandlung höher oder geringer ist als am Behandlungsende.

Die Vorrichtung wirkt besonders gut, wenn Dialysatoren verwendet werden, die als High- Flux Dialysatoren bezeichnet werden. Als High- Flux Dialysatoren werden Filter bezeichnet, die eine Ultrafiltrationrate von 20-70 ml/m² * mmHg * h in Humanblut aufweisen.

Besonders wirkt die erfindungsgemäße Vorrichtung in Verbindung mit einem sog. Mid-cut-off Dialysator. Ein solcher Dialysator ist beispielsweise in der WO 2015/118046 A beschrieben, auf die hier insoweit Bezug genommen wird. Solche Dialysatoren weisen eine unkontrollierte Rückspülung von Dialysat in den Blutkreislauf auf, was durch die internen Druckverhältnisse im Dialysator zustande kommt. Diese Dialysatoren dürfen nicht im HDF- Modus betrieben werden, da ansonsten der Verlust an Albumin bedeutend zu hoch ausfallen würde.

Mit Hilfe der erfindungsgemäßen Vorrichtung kann nun mit einer "normalen" Maschine im "kontrollierten Substituatmodus" betrieben werden. Insbesondere kann durch geschickte Programmwahl die Substituatmenge auf Volumina zwischen 2 und 15 l / Behandlung, bevorzugt auf Volumina zwischen 5 und 12 l / Behandlung, weiter bevorzugt auf Volumina zwischen 5 und 10 l / Behandlung begrenzt werden.

## Patentansprüche

1. Dialysegerät mit einem Dialysator (D) und mit einer ersten Bilanzkammer (A) und mit einer zweiten Bilanzkammer (B), von denen jede wenigstens zwei durch eine bewegliche Wand (W) voneinander getrennte erste (100, 102) und zweite Bilanzkammerhälften (200, 202) aufweist, wobei jede erste Bilanzkammerhälfte (100, 102) mit jeweils einem ersten Zulauf (Z1, Z3) und mit jeweils einem ersten Ablauf (A1, A3) versehen ist, wobei jede zweite Bilanzkammerhälfte (200, 202) mit jeweils einem zweiten Zulauf (Z2, Z4) und mit jeweils einem zweiten Ablauf (A2, A4) versehen ist, wobei die Zu- und die Abläufe (A1-A4, Z1-Z4) jeweils mit Ventilen (1.1-1.4; 2.1-2.4) versehen sind, die ausgebildet sind, den jeweiligen Zulauf (Z1-Z4) bzw. Ablauf (A1-A4) zu schließen oder zu öffnen, wobei die ersten Zuläufe (Z1; Z3) der ersten Bilanzkammerhälften (100; 102) mit einer Quelle frischen Dialysats und die ersten Abläufe (A1; A3) der ersten Bilanzkammerhälften (100; 102) mit einem Dialysatorzulauf (DZ) in Fluidverbindung stehen und wobei die zweiten Zuläufe (Z2; Z4) der zweiten Bilanzkammerhälften (200; 202) mit einem Dialysatorablauf (DA) in Fluidverbindung stehen und die zweiten Abläufe (A2; A4) der zweiten Bilanzkammerhälften (200; 202) mit einem Drain (1) in Fluidverbindung stehen, **dadurch gekennzeichnet, dass** das Dialysegerät eine dritte Bilanzkammer (C) aufweist, die über zwei Bilanzkammerhälften (104; 204) verfügt, die durch eine bewegliche Wand (W) voneinander getrennt sind, wobei jede der Bilanzkammerhälften (104; 204) jeweils über einen Zulauf (Z5; Z6) und über einen Ablauf (A5; A6) verfügt, die jeweils mit Ventilen (V1-V4) versehen sind, die ausgebildet sind, den jeweiligen Zulauf (Z5; Z6) bzw. Ablauf (A5; A6) zu schließen oder zu öffnen, wobei die beiden Zuläufe (Z5; Z6) mit einer Quelle frischen Dialysats in Fluidverbindung stehen und wobei die beiden Abläufe (A5; A6) mit dem Dialysatorzulauf (DZ) in Fluidverbindung stehen.

2. Dialysegerät nach Anspruch 1, **dadurch gekennzeichnet, dass** die beiden Zuläufe (Z5; Z6) der dritten Bilanzkammer (C) mit derselben Quelle frischen Dialysats in Fluidverbindung stehen, wie die ersten Zuläufe (Z1; Z3) der ersten und zweiten Bilanzkammer (A; B).

3. Dialysegerät nach Anspruch 1 oder 2, **dadurch gekennzeichnet, dass** die beiden Abläufe (A5; A6) der dritten Bilanzkammer (C) mit derselben, zum Dialysatorzulauf (DZ) führenden Leitung (30) in Fluidverbindung stehen, wie die ersten Abläufe (A2; A4) der ersten und der zweiten Bilanzkammer (A; B).

4. Dialysegerät nach einem der vorhergehenden Ansprüche, **dadurch gekennzeichnet, dass** das Gerät keine Substituatpumpe zur Förderung einer Substitutionslösung in das Blut des Patienten aufweist.

5. Dialysegerät nach einem der vorhergehenden Ansprüche, **dadurch gekennzeichnet, dass** die Bilanzkammerhälften (104; 204) der dritten Bilanzkammer (C) ein kleineres Volumen aufweisen als die Bilanzkammerhälften der ersten und der zweiten Bilanzkammer (A; B).

6. Dialysegerät nach einem der vorhergehenden Ansprüche, **dadurch gekennzeichnet, dass** stromaufwärts und/oder stromabwärts der dritten Bilanzkammer (C) ein Drosselorgan angeordnet ist, das ausgebildet ist, den Zustrom und/oder den Abstrom frischen Dialysats aus der dritten Bilanzkammer (C) zu drosseln.

7. Dialysegerät nach einem der vorhergehenden Ansprüche, **dadurch gekennzeichnet, dass** eine Steuerung vorgesehen ist, die ausgebildet ist, die Zufuhr frischen Dialysats zum Dialysator (D) aus der dritten Bilanzkammer (C) über die Behandlungsdauer zeitlich gleichmäßig oder zeitlich ungleichmäßig verteilt durchzuführen.

8. Dialysegerät nach einem der vorhergehenden Ansprüche, **dadurch gekennzeichnet, dass** es sich bei dem Dialysator (D) um einen High-Flux-Dialysator oder um einen Mid-cut-off-Dialysator handelt.

9. Steuereinheit konfiguriert zur Durchführung eines Verfahrens zum Betreiben eines Bilanzkammersystems eines Dialysegerätes mit einer ersten Bilanzkammer (A) und mit einer zweiten Bilanzkammer (B), von denen jede wenigstens zwei durch eine bewegliche Wand (W) voneinander getrennte erste (100, 102) und zweite Bilanzkammerhälften (200, 202) aufweist, wobei jede erste Bilanzkammerhälfte (100, 102) mit jeweils einem ersten Zulauf (Z1, Z3) und mit jeweils einem ersten Ablauf (A1, A3) versehen ist, wobei jede zweite Bilanzkammerhälfte (200, 202) mit jeweils einem zweiten Zulauf (Z2, Z4) und mit jeweils einem zweiten Ablauf (A2, A4) versehen ist, wobei die Zu- und die Abläufe (A1-A4, Z1-Z4)jeweils mit Ventilen (1.1-1.4; 2.1-2.4) versehen sind, die ausgebildet sind, den jeweiligen Zulauf (Z1-Z4) bzw. Ablauf (A1-A4) zu schließen oder zu öffnen, wobei die ersten Zuläufe (Z1; Z3) der ersten Bilanzkammerhälften (100; 102) mit einer Quelle frischen Dialysats und die ersten Abläufe (A1; A3) der ersten Bilanzkammerhälften (100; 102) mit einem Dialysatorzulauf (DZ) in Fluidverbindung stehen und wobei die zweiten Zuläufe (Z2; Z4) der zweiten Bilanzkammerhälften (200; 202) mit einem Dialysatorablauf (DA) in Fluidverbindung stehen und die zweiten Abläufe (A2; A4) der zweiten Bilanzkammerhälften (200; 202) mit einem Drain (1) in Fluidverbindung stehen, **dadurch gekennzeichnet, dass** das Dialysegerät eine dritte Bilanzkammer (C) aufweist, die über zwei Bilanzkammerhälften (104; 204) verfügt, die durch eine bewegliche Wand (W) voneinander getrennt sind, wobei jede der Bilanzkammerhälften (104; 204) jeweils über einen Zulauf (Z5; Z6) und über einen Ablauf (A5; A6) verfügt, die jeweils mit Ventilen (V1-V4) versehen sind, wobei die beiden Zuläufe (Z5; Z6) mit einer Quelle frischen Dialysats in Fluidverbindung stehen und wobei die beiden Abläufe (A5; A6) mit dem Dialysatorzulauf (DZ) in Fluidverbindung stehen, wobei die Ventile (V1-V4) so betrieben werden, dass frisches Dialysat von der dritten Bilanzkammer (C) zu dem Dialysatorzulauf (DZ) gefördert wird.

10. Steuereinheit nach Anspruch 9, **dadurch gekennzeichnet, dass** die Ventile (V1-V4) der dritten Bilanzkammer so betrieben werden, dass sich ein kontinuierlicher Fluss frischen Dialysats aus der dritten Bilanzkammer (C) zu dem Dialysatorzulauf (DZ) ergibt.

11. Steuereinheit nach Anspruch 9 oder 10, **dadurch gekennzeichnet, dass** die dritte Bilanzkammer (C) kontinuierlich oder diskontinuierlich betrieben wird.

12. Steuereinheit nach einem der Ansprüche 9 bis 11, **dadurch gekennzeichnet, dass** die Zufuhr frischen Dialysats zur dritten Bilanzkammer (C) und/oder die Abfuhr frischen Dialysats aus der dritten Bilanzkammer (C) zu dem Dialysatorzulauf gedrosselt wird.

13. Steuereinheit nach einem der Ansprüche 9 bis 12, **dadurch gekennzeichnet, dass** die dritte Bilanzkammer (C) so betrieben wird, dass die Förderrate frischen Dialysats aus der dritten Bilanzkammer (C) zu dem Dialysatorzulauf über die Behandlungsdauer konstant ist oder variiert.

14. Steuereinheit nach einem der Ansprüche 9 bis 13, **dadurch gekennzeichnet, dass** zur Zufuhr von Substituat zum Patienten keine Substituatpumpe verwendet wird.

## Claims

1. A dialysis machine having a dialyzer (D) and having a first balancing chamber (A) and having a second balancing chamber (B) of which each has at least two first (100, 102) and second balancing chamber halves (200, 202) separated from one another by a movable wall (W), wherein each first balancing chamber half (100, 102) is provided with a respective first inflow (Z1, Z3) and with a respective first outflow (A1, A3), wherein each second balancing chamber half (200, 202) is provided with a respective second inflow (Z2, Z4) and with a respective second outflow (A2, A4), wherein the inflows and outflows (A1-A4, Z1-Z4) are each provided with valves (1.1-1.4; 2.1-2.4) that are configured to close or to open the respective inflow (Z1-Z4) or outflow (A1-A4), wherein the first inflows (Z1; Z3) of the first balancing chamber halves (100; 102) are in fluid communication with a source of fresh dialyzate and the first outflows (A1; A3) of the first balancing chamber halves (100; 102) are in fluid communication with a dialyzer inflow (DZ), and wherein the second inflows (Z2; Z4) of the second balancing chamber halves (200; 202) are in fluid communication with a dialyzer outflow (DA) and the second outflows (A2; A4) of the second balancing chamber halves (200; 202) are in fluid communication with a drain (1), **characterized in that** the dialysis machine has a third balancing chamber (C) is provided that has two balancing chamber halves (104; 204) that are separated from one another by a movable wall (W), with each of the balancing chamber halves (104; 204) having a respective inflow (Z5; Z6) and a respective outflow (A5; A6) that are each provided with valves (V1-V4) that are configured to close or to open the respective inflow (Z5; Z6) or outflow (A5; A6), with the two inflows (Z5; Z6) being in fluid communication with a source of fresh dialyzate and with the two outflows (A5; A6) being in fluid communication with the dialyzer inflow (DZ).

2. Dialysis machine in accordance with claim 1, **characterized in that** the two inflows (Z5; Z6) of the third balancing chamber (C) are in fluid communication with the same source of fresh dialyzate as the first inflows (Z1; Z3) of the first and second balancing chambers (A; B).

3. Dialysis machine in accordance with claim 1 or claim 2, **characterized in that** the two outflows (A5; A6) of the third balancing chamber (C) are in fluid communication with the same line (30) leading to the dialyzer inflow (DZ) as the first outflows (A2; A4) of the first and second balancing chambers (A; B).

4. Dialysis machine in accordance with one of the preceding claims, **characterized in that** the machine does not have any substituate pump for conveying a substitution solution into the blood of the patient.

5. Dialysis machine in accordance with one of the preceding claims, **characterized in that** the balancing chamber halves (104; 204) of the third balancing chamber (C) have a smaller volume than the balancing chamber halves of the first and second balancing chambers (A; B).

6. Dialysis machine in accordance with one of the preceding claims, **characterized in that** a throttle member is arranged upstream and/or downstream of the third balancing chamber (C), said throttle member being configured to throttle the inward flow and/or the outward flow of fresh dialyzate from the third balancing chamber (C).

7. Dialysis machine in accordance with one of the preceding claims, **characterized in that** a control is provided that is configured to carry out the supply of fresh dialyzate to the dialyzer (D) from the third balancing chamber (C) evenly in time or unevenly in time over the treatment duration.

8. Dialysis machine in accordance with one of the preceding claims, **characterized in that** the dialyzer (D) is a high-flux dialyzer or a medium cut-off dialyzer.

9. Control unit, configured for performing a method of operating a balancing chamber system of a dialysis machine having a first balancing chamber (A) and having a second balancing chamber (B) of which each has at least two first (100, 102) and second balancing chamber halves (200, 202) separated from one another by a movable wall (W), wherein each first balancing chamber half (100, 102) is provided with a respective first inflow (Z1, Z3) and with a respective first outflow (A1, A3), wherein each second balancing chamber half (200, 202) is provided with a respective second inflow (Z2, Z4) and with a respective second outflow (A2, A4), wherein the inflows and outflows (A1-A4, Z1-Z4) are each provided with valves (1.1-1.4; 2.1-2.4) that are configured to close or to open the respective inflow (Z1-Z4) or outflow (A1-A4), wherein the first inflows (Z1; Z3) of the first balancing chamber halves (100; 102) are in fluid communication with a source of fresh dialyzate and the first outflows (A1; A3) of the first balancing chamber halves (100; 102) are in fluid communication with a dialyzer inflow (DZ), and wherein the second inflows (Z2; Z4) of the second balancing chamber halves (200; 202) are in fluid communication with a dialyzer outflow (DA) and the second outflows (A2; A4) of the second balancing chamber halves (200, 202) are in fluid communication with a drain (1), **characterized in that** the dialysis machine has a third balancing chamber (C) that has two balancing chamber halves (104; 204) that are separated from one another by a movable wall (W), with each of the balancing chamber halves (104; 204) having a respective inflow (Z5; Z6) and a respective outflow (A5; A6) that are each provided with valves (V1-V4), with the two inflows (Z5; Z6) being in fluid communication with a source of fresh dialyzate and with the two outflows (A5; A6) being in fluid communication with the dialyzer inflow (DZ), with the valves (V1-V4) being operated such that fresh dialyzate is conveyed from the third balancing chamber (C) to the dialyzer inflow (DZ).

10. Control unit in accordance with claim 9, **characterized in that** the valves (V1-V4) of the third balancing chamber are operated such that a continuous flow of fresh dialyzate results from the third balancing chamber (C) to the dialyzer inflow (DZ).

11. Control unit in accordance with claim 9 or claim 10, **characterized in that** the third balancing chamber (C) is operated continuously or discontinuously.

12. Control unit in accordance with one of the claims 9 to 11, **characterized in that** the supply of fresh dialyzate to the third balancing chamber (C) and/or the removal of fresh dialyzate from the third balancing chamber (C) to the dialyzer inflow is throttled.

13. Control unit in accordance with one of the claims 9 to 12, **characterized in that** the third balancing chamber (C) is operated such that the conveying rate of fresh dialyzate from the third balancing chamber (C) to the dialyzer inflow is constant or varied over the treatment duration.

14. Control unit in accordance with one of the claims 9 to 13, **characterized in that** no substituate pump is used to supply substituate to the patient.

## Revendications

1. Appareil de dialyse avec un dialyseur (D) et avec une première chambre de bilan (A) et avec une deuxième chambre de bilan (B), dont chacune présente au moins deux premières (100, 102) et deuxièmes moitiés de chambre de bilan (200, 202) séparées l'une de l'autre par une paroi mobile (W), chaque première moitié de chambre de bilan (100, 102) étant pourvue respectivement d'une première entrée (Z1, Z3) et d'une première sortie (A1, A3), chaque deuxième moitié de chambre de bilan (200, 202) étant pourvue respectivement d'une deuxième entrée (Z2, Z4) et d'une deuxième sortie (A2, A4), les entrées et les sorties (A1-A4, Z1-Z4) étant pourvues de soupapes (1.1-1.4 ; 2.1-2.4) qui sont réalisées pour fermer er ouvrir l'entrée (Z1-Z4) et la sortie (A1-A2) respectives, les premières entrées (Z1 ; Z3) des premières moitiés de chambre de bilan (100 ; 102) étant en communication fluidique avec une source de dialysat frais et les premières sorties (A1 ; A3) des premières moitiés de chambre de bilan (100 ; 102) étant en communication fluidique avec une entrée de dialyseur (DZ) et les deuxièmes entrées (Z2 ; Z4) des deuxièmes moitiés de chambre de bilan (200 ; 202) étant en communication fluidique avec une sortie de dialyseur (DA) et les deuxièmes sorties (A2 ; A4) des deuxièmes moitiés de chambre de bilan (200 ; 202) étant en communication fluidique avec un drain (1), **caractérisé en ce que** l'appareil de dialyse présente une troisième chambre de bilan (C) qui dispose de deux moitiés de chambre de bilan (104 ; 204) qui sont séparées l'une de l'autre par une paroi mobile (W), chacune des moitiés de chambre de bilan (104 ; 204) disposant respectivement d'une entrée (Z5 ; Z6) et d'une sortie (A5 ; A6) qui sont respectivement pourvues de soupapes (V1-V4) qui sont réalisées pour ouvrir et pour fermer l'entrée (Z5 ; Z6) et la sortie (A5 ; A6) respectives, les deux entrées (Z5 ; Z6) étant en communication fluidique avec une source de dialysat frais et les deux sorties (A5 ; A6) étant en communication fluidique avec l'entrée de dialyseur (DZ).

2. Appareil de dialyse selon la revendication 1, **caractérisé en ce que** les deux entrées (Z5 ; Z6) de la troisième chambre de bilan (C) sont en communication fluidique avec la même source de dialysat frais que les premières entrées (Z1 ; Z3) de la première et de la deuxième chambre de bilan (A ; B).

3. Appareil de dialyse selon la revendication 1 ou 2, **caractérisé en ce que** les deux sorties (A5 ; A6) de la troisième chambre de bilan (C) sont en communication fluidique avec la même conduite (30) menant à l'entrée de dialyseur (DZ) que les premières sorties (A2 ; A4) de la première et de la deuxième chambre de bilan (A ; B).

4. Appareil de dialyse selon l'une quelconque des revendications précédentes, **caractérisé en ce que** l'appareil ne présente pas de pompe de substitut pour transporter une solution de substitution dans le sang du patient.

5. Appareil de dialyse selon l'une quelconque des revendications précédentes, **caractérisé en ce que** les moitiés de chambre de bilan (104 ; 204) de la troisième chambre de bilan (C) présentent un volume inférieur à celui des moitiés de chambre de bilan de la première et de la deuxième chambre de bilan (A ; B).

6. Appareil de dialyse selon l'une quelconque des revendications précédentes, **caractérisé en ce qu'**un organe d'étranglement est agencé en amont et/ou en aval de la troisième chambre de bilan (C), lequel est réalisé pour étrangler le flux entrant et/ou le flux sortant de dialysat frais de la troisième chambre de bilan (C).

7. Appareil de dialyse selon l'une quelconque des revendications précédentes, **caractérisé en ce qu'**il est prévu une commande qui est réalisée pour effectuer l'amenée de dialysat frais au dialyseur (D) à partir de la troisième chambre de bilan (C) de manière régulière dans le temps ou irrégulière dans le temps pendant la durée du traitement.

8. Appareil de dialyse selon l'une quelconque des revendications précédentes, **caractérisé en ce que** le dialyseur (D) consiste en un dialyseur à haut débit ou un dialyseur Mid-Cut-Off.

9. Unité de commande configurée pour effectuer un procédé pour l'exploitation d'un système de chambre de bilan d'un appareil de dialyse avec une première chambre de bilan (A) et avec une deuxième chambre de bilan (B), dont chacune présente au moins deux premières (100, 102) et deuxièmes moitiés de chambre de bilan (200, 202) séparées l'une de l'autre par une paroi mobile (W), chaque première moitié de chambre de bilan (100, 102) étant pourvue d'une première entrée respective (Z1, Z3) et d'une première sortie respective (A1, A3), chaque deuxième moitié de chambre de bilan (200, 202) étant pourvue d'une deuxième entrée respective (Z2, Z4) et d'une deuxième sortie respective (A2, A4), les entrées et les sorties (A1-A4, Z1-Z4) étant respectivement équipées de soupapes (1.1-1.4 ; 2.1-2.4) qui sont réalisées pour ouvrir et pour fermer l'entrée (Z1-Z4) et la sortie (A1-A4) respectives, les premières entrées (Z1 ; Z3) des premières moitiés de chambre de bilan (100 ; 102) étant en communication fluidique avec une source de dialysat frais et les premières sorties (A1 ; A3) des premières moitiés de chambre de bilan (100 ; 102) étant en communication fluidique avec une entrée de dialyseur (DZ) et les deuxièmes entrées (Z2 ; Z4) des deuxièmes moitiés de chambre de bilan (200 ; 202) étant en communication fluidique avec une sortie de dialyseur (DA) et les deuxièmes sorties (A2 ; A4) des deuxièmes moitiés de chambre de bilan (200 ; 202) étant en communication fluidique avec un drain (1), **caractérisé en ce que** l'appareil de dialyse présente une troisième chambre de bilan (C) qui dispose de deux moitiés de chambre de bilan (104 ; 204), qui sont séparées l'une de l'autre par une paroi mobile (W), chacune des moitiés de chambre de bilan (104 ; 204) disposant respectivement d'une entrée (Z5 ; Z6) et d'une sortie (A5 ; A6) qui sont pourvues respectivement de soupapes (V1-V4), les deux entrées (Z5 ; Z6) étant en communication fluidique avec une source de dialysat frais et les deux sorties (A5 ; A6) étant en communication fluidique avec l'entrée de dialyseur (DZ), les soupapes (V1-V4) étant exploitées pour transporter du dialysat frais de la troisième chambre de bilan (C) à l'entrée de dialyseur (DZ).

10. Unité de commande selon la revendication 9, **caractérisée en ce que** les soupapes (V1-V4) de la troisième chambre de bilan sont exploitées de manière à obtenir un flux continu de dialysat frais depuis la troisième chambre de bilan (C) vers l'entrée de dialyseur (DZ).

11. Unité de commande selon la revendication 9 ou 10, **caractérisée en ce que** la troisième chambre de bilan (C) est exploitée de manière continue ou discontinue.

12. Unité de commande selon l'une quelconque des revendications 9 à 11 , **caractérisée en ce que** l'amenée de dialysat frais à la troisième chambre de bilan (C) et/ou l'évacuation de dialysat frais depuis la troisième chambre d'équilibrage (C) vers l'entrée de dialyseur sont étranglées.

13. Unité de commande selon l'une quelconque des revendications 9 à 12, **caractérisée en ce que** la troisième chambre de bilan (C) est exploitée de telle sorte que le débit de dialysat frais de la troisième chambre de bilan (C) vers l'entrée de dialyseur est constant ou varie pendant la durée du traitement.

14. Unité de commande selon l'une quelconque des revendications 9 à 13, **caractérisée en ce qu'**aucune pompe de substitut n'est utilisée pour amener un substitut au patient.
